**Europäisches Patentamt**

**European Patent Office**

**Office européen des brevets**

(19)

(11) Veröffentlichungsnummer: **0 018 022 B1**

# EUROPÄISCHE PATENTSCHRIFT

(12)

(45) Veröffentlichungstag der Patentschrift:
**12.01.83**

(21) Anmeldenummer: **80200203.0**

(22) Anmeldetag: **03.03.80**

(51) Int. Cl.³: **B 01 J 27/16, C 07 C 29/04**

(54) **Verfahren zur Herstellung eines Katalysators für die Hydratation von Olefinen zu Alkoholen.**

(30) Priorität: **05.03.79 DE 2908491**

(43) Veröffentlichungstag der Anmeldung:
**29.10.80 Patentblatt 80/22**

(45) Bekanntmachung des Hinweises auf die Patenterteilung:
**12.01.83 Patentblatt 83/2**

(84) Benannte Vertragsstaaten:
**BE DE FR GB IT NL**

(56) Entgegenhaltungen: '
**US-A-3 704 329**
**UA-A-3 784 614**
**US-A-3 862 249**

(73) Patentinhaber: **CHEMISCHE WERKE HÜLS AG,**
**Postfach 1320, D-4370 Marl 1 (DE)**

(72) Erfinder: **Sommer, August, Dr., Birnenbruchstrasse 10,**
**D-4690 Herne 1 (DE)**
Erfinder: **Brücker, Rainer, Dr., Unterspredey 65,**
**D-4620 Castrop-Rauxel (DE)**

(74) Vertreter: **Steil, Hanna, Dipl.-Chem. et al, RSP**
**PATENTE-PB 40 Holsterhauser**
**Strasse 160 Postfach 2840, D-4690 Herne 2 (DE)**

Verfahren zur Herstellung eines Katalysators für die Hydratation von Olefinen zu Alkoholen

Es ist bekannt, dass sich Olefine in der Gasphase bei erhöhten Drücken mit Wasserdampf zu Alkoholen umsetzen lassen. Besondere technische Bedeutung haben solche Verfahren bei der Herstellung von Ethylalkohol aus Ethylen und Isopropylalkohol aus Propylen erlangt. Die Synthese dieser Alkohole wird in Gegenwart von Katalysatoren durchgeführt, und zwar dient in aller Regel als Katalysator Phosphorsäure, die auf Trägeren aufgebracht ist.

Bekannt sind Trägermaterialien entweder auf der Basis reiner Kieselsäure (z.B. Kieselgur oder Kieselgel) oder auf der Basis von Kieselsäure mit mehr oder weniger grossem Tonerdegehalt, wie z.B. kalzinierte Diatomeenerde, deren Struktur durch Ton oder tonartige Stoffe zusammengehalten wird.

Bei den Trägern auf Basis reiner Kieselsäure ist die Festigkeit über längere Standzeiten problematisch. Die tonerdehaltigen Materialien zeichnen sich zwar durch eine bessere mechanische Festigkeit aus, sie haben jedoch bei zu hohem Tonerdegehalt den Nachteil, dass während der Reaktion das Aluminiumoxid durch die Einwirkung der Phosphorsäure herausgelöst wird, wodurch es in den nachgeschalteten Apparaten zu störenden Belegungen kommt.

Es ist auch möglich geworden, auf der Basis grobporiger Kieselgele Träger für Phosphorsäure mit hoher Hydratationsaktivität und ausreichender mechanischer Festigkeit zu entwickeln, z.B. DE-A Nr. 2625705 und DE-A Nr. 2719055. Allerdings verbleibt als Nachteil dieser Träger auf der Basis amorpher Kieselsäure, dass bei längerem Aussetzen gegen die Bedingungen der Hydratationsreaktion die amorphe Kieselsäure teilweise zu Cristobalit und Quarz kristallisiert, was mit starker Verminderung der spezifischen Oberfläche und damit der katalytischen Aktivität, und zwar irreversibel, verbunden ist, sowie mit einer Abnahme der mechanischen Festigkeit.

In DE-C Nr. 1156772 wurde ein Verfahren zur Herstellung eines tonerdehaltigen Trägers für die bei der Olefinhydratation als Katalysator verwendete Phosphorsäure beschrieben, in dem geformte Kontaktkörper aus mineralischen Tonerdesilikaten, insbesondere handelsübliche bentonithaltige Kontaktkörper für Hydrierungen und Dehydrierungen, derart mit Mineralsäure behandelt werden, dass der Aluminiumoxidgehalt vorzugsweise auf zwischen 1 und 5 Gew.% abgesenkt wird. Dieses Material weist sowohl die erforderliche mechanische Festigkeit auf als auch einen ausreichend niedrigen Restaluminiumoxidgehalt, um eine Belegung nachgeschalteter Apparate durch mit Phosphorsäure herausgelöste Bestandteile zu vermeiden. Im Laufe landjähriger Erfahrungen beim Einsatz handelsüblicher bentonithaltiger Kontaktkörper für Hydrierungen und Dehydrierungen wurde nun die Beobachtung gemacht, dass es bei der Herstellung der handelsüblichen Kontaktkörper für den Verwendungszweck der Hydrierung und Dehydrierung nicht in dem Masse wie zur Herstellung des Trägermaterials für Phosphorsäure erforderlich ist, eine Vorauswahl hinsichtlich des Rohstoffs zu treffen. So weisen die durch Säurebehandlung und Imprägnierung mit Phosphorsäure hergestellten Hydratationskatalysatoren aus den handelsüblichen bentonithaltigen Kontaktkörpern stark unterschiedliche Aktivitäten auf, was zu starken Schwankungen der Anlagenkapazität bei vorliegendem Reaktorvolumen in kontinuierlich arbeitenden Syntheseanlagen führte.

Da inzwischen gefunden wurde, dass durch kontinuierliche Einspritzung der ausgetragenen Phosphorsäuremenge (DE-A Nr. 2658946) die Katalysatorlebensdauer beträchtlich verlängert wird und somit entsprechende Anforderungen auch an die Lebensdauer des Trägers zu stellen sind, scheidet die Verwendung solcher Träger aus, bei denen unter Reaktionsbedingungen eine Verminderung der katalytischen Aktivität z.B. Kristallisationen auf irreversible Weise ablaufen und/oder die mechanische Festigkeit im Laufe der Zeit abnimmt.

Es wurde nun überraschenderweise gefunden, dass aus bestimmten Tonen hergestellte Tonmineralien Träger für Hydratationskatalysatoren gleichbleibend hoher katalytischer Aktivität erhalten werden können, wenn durch sorgfältige Auswahl des Rohstoffs Vorsorge dafür getroffen wird, dass das Material in hohem Masse aus Montmorillonit besteht und nicht mehr als 5 g K (berechnet als $K_2O$)/kg Ton enthält.

Es wurde weiterhin gefunden, dass eine Abhängigkeit der katalytischen Aktivität (ausgedrückt in maximal möglicher Alkoholerzeugung pro Volumen Katalysatorschüttung und Zeit) von der spezifischen Oberfläche des Trägers nach Tränken mit Phosphorsäure und Einstellen der unter Betriebsbedingungen vorliegenden Phosphorsäurekonzentration nach der zweiten Säurebehandlung besteht. Diese Abhängigkeit zeigt sich darin, dass sich beste katalytische Aktivitätswerte dann ergeben, wenn die spezifische Oberfläche nach der zweiten Säurebehandlung, aber vor Imprägnierung, um 200 m²/g liegt.

Gegenstand der Erfindung ist daher ein Verfahren zur Herstellung eines Katalysators für die Hydratation von Olefinen mit 2 bis 3 C-Atomen zu den entsprechenden Alkoholen, durch Behandlung eines geformten Trägers aus montmorillonithaltigem Ton mit Säure, bis er einen $Al_2O_3$-Gehalt von 1 bis 5% aufweist, Tränken des Trägers mit Phosphorsäure und Trocknen, dadurch gekennzeichnet, dass man als Träger einen Ton verwendet, der mit nicht mehr als 3% Begleitmineralien, wie Quarz, Feldspat und Glimmer, verunreinigt ist und bis 0,5% $K_2O$ enthalten darf, diesen Ton vor der Verformung bereits mit Säure behandelt, bis er einen $Al_2O_3$-Gehalt von 13 bis 18 Gew.% aufweist (1.Stufe), gegebenenfalls durch Zugabe von gefällter Tonerde den $Al_2O_3$-Gehalt auf 16 bis

18 Gew.% einstellt, wobei sich eine Oberfläche von 200 bis 400 m²/g, bevorzugt 240 bis 300 m²/g, ergibt, dann bei einem Gesamtwassergehalt von 20 bis 35% durch Pressen formt, bei 500 bis 800° C calciniert und anschliessend in bekannter Weise unter nochmaliger Säurebehandlung (2.Stufe) weiterverarbeitet.

Weitere Ausgestaltungen des Verfahrens ergeben sich aus den Unteransprüchen. Gegenstand der Erfindung ist weiterhin ein Katalysator zur Hydratation von Olefinen, wie vorstehend beschrieben. Untersucht man die spezifische Oberfläche des erst einmal säurebehandelten Trägers auf Montmorillonitbasis, so stellt man fest, dass sie zwischen 200 und 400 m²/g liegt, bevorzugt 240 bis 300 m²/g.

Der zweimal säurebehandelte, aber nicht mit Phosphorsäure imprägnierte Träger zeigt eine spezifische Oberfläche von 150 bis 250 m²/g, bevorzugt 180 bis 220 m²/g.

Darüber hinaus wurde auch eine gewisse Abhängigkeit der katalytischen Aktivität von der Schüttkörper-Oberfläche festgestellt, so dass eine höhere Aktivität mit kleineren als bisher üblichen (5 mm) Kugel- oder Zylinderdurchmessern, nämlich 2 bis 5 mm, erreicht werden konnte.

Die Besonderheit des Tonminerals Montmorillonit mit der Summenformel $Al_2 (Si_4O_{10}) (OH)_2$ besteht darin, dass eine Schicht von $Al_2O_3$-Oktaedern von 2 Schichten $SiO_2$-Tetraedern jeweils eingeschlossen wird, wobei Restladungen durch Protonen abgesättigt sind. Durch die thermische Säurebehandlung kann das Aluminium zu einem grossen Teil herausgelöst werden, wobei zunächst eine Vergrösserung der Oberfläche eintritt, solange nur von dem Aluminium hinterlassene negative Ladungen an den verbleibenden $SiO_2$-Tetraedern durch Protonen abgesättigt werden, die Montmorillonit-Schichtenstruktur jedoch erhalten bleibt.

Erst wenn bei weiterem Herauslösen von Aluminium die Montmorillonitstruktur zerstört wird, verringert sich die Oberfläche wieder. Dieser Bereich muss bei den Trägern für phosphorsäurehaltig Hydratationskatalysatoren jedoch erreicht werden, um die Abscheidung von Aluminiumsalzen der Phosphorsäure in nachgeschalteten Apparaturen zu vermeiden, die sich bei einem zu hohen Restaluminiumgehalt mit Phosphorsäure bilden würden.

Bei den Mineralen Glimmer und Feldspat, die zu der Gruppe der Alumosilikate gehören, liegen nicht wie beim Montmorillonit ausschliesslich kationische Schichten von Aluminium vor, sondern Aluminium ist ganz (Feldspat) oder teilweise (Glimmer) in das $SiO_2$-Tetraedergitter eingebaut, wobei überschüssige negative Ladungen überwiegend durch Kaliumkationen abgesättigt sind. Bei der Säurebehandlung von Glimmer K $Al_2$ (Al $Si_3 O_{10}$) $(OH)_2$ und Feldspat K (Al $Si_3O_8$) werden zwar die Kationen Kalium (beim Glimmer auch das Al ausserhalb der Klammer), nicht jedoch das in die Silikatstruktur eingebaute Aluminium durch Protonen, ersetzt. Es entfällt also im Vergleich zum Montmorillonit die Auflockerung der Kristalistruktur mit Vergrösserung der Oberfläche bei der Säurebehandlung. Andere Minerale der Montmorillonitgruppe, die kein Kalium enthalten und auch das gleiche Kristallgitter wie der Montmorillonit mit nur einigen durch Fremdionen verursachten Fehlstellen besitzen, sind in diese Betrachtungen eingeschlosse, da sie sich bei der zweistufigen Säurebehandlung völlig analog dem reinen Montmorillonit verhalten. Es sind dies Saponit mit Magnesium anstelle von Aluminium im Oktaeder, Hectorit mit zusätzlich Lithium zum Magnesium, Nontronit mit Eisen und Saukonit mit Zink an dieser Stelle sowie Beidellit mit Aluminium im Tetraedergitter des Siliciums.

Eine optimale Oberfläche der geformten Kontaktkörper ist also beim Einsatz solcher Rohtone zu erwarten, die einen maximalen Montmorillonitgehalt aufweisen, weil nur dieses Mineral zur Vergrösserung der Oberfläche nach Säurebehandlung beiträgt. Gemeinsam ist den beiden Mineralien Glimmer und Feldspat ohne Beitrag zur Oberflächenvergrösserung ihr Kaliumgehalt, der durch Säurebehandlung extrahiert wird. Über die Kaliumbestimmung des Extraktes mit 15 bis 20%iger Salzsäure bei 80 bis 90° C kann also eine Aussage darüber gemacht werden, ob ein bestimmter Rohstoff für die Herstellung von Trägermaterial für den Hydratationskatalysator geeignet ist.

Rein rechnerisch enthält die Trockensubstanz im Glimmer 12% $K_2O$, im Feldspat 17% $K_2O$. Wenn sich aus der durch Behandlung mit Salzsäure extrahierbaren Kaliummenge ein $K_2O$-Gehalt im Rohton (auf Trockensubstanz bezogen) von unter 0,5% ergibt, kann davon ausgegangen werden, dass eine spezifische Oberfläche von 200 bis 400 m²/g erhalten wird, und dass der geformte Kontaktkörper nach der zweiten Säurebehandlung eine genügend grosse spezifische Oberfläche (150 bis 250 m²/g) besitzt, um über optimale Eigenschaften als Träger für Hydratationskatalysator zu verfügen.

Handelsübliche hochaktive Bleicherde ist aus entsprechendem Ton durch Säurebehandlung wie in der ersten Stufe hergestellt worden, bei der der Aluminiumgehalt auf 13 bis 18 Gew.% abgesenkt wird. Sie kann daher genau wie ein Ton der genannten Eigenschaften nach der ersten Säurebehandlungsstufe eingesetzt werden.

*Beispiel 1:*

Ein gemahlener natürlicher Rohton, der aufgrund einer Laboruntersuchung so ausgewählt wurde, dass bei einer einstündigen Behandlung mit 20%iger Salzsäure bei 82° C nicht mehr als 5 g $K_2O$/kg eingesetzte Trockensubstanz extrahiert werden, wurde 1 h lang mit 20%iger Salzsäure auf 82° C erhitzt, wobei die Säuremenge so bemessen wurde, dass auf 1 kg Ton 8,4 mol HCl kamen, säurefrei gewaschen und getrocknet. Dabei wurde ein Material mit einem Rest Aluminiumoxidgehalt von 16% und einer spezifischen Oberfläche von 310 m²/g erhalten.

Nach Anfeuchten mit 25% Wasser auf die Gesamtmenge bezogen (das ist Zugabe von 33% der Trockensubstanz als Wasser) wurde das Material in zylindrische Form von 4 mm Durchmesser und 4 mm Höhe gepresst und verfestigt durch 3 h langes Erhitzen auf 600° C.

Die so erhaltenen geformten Kontaktkörper wurden insgesamt zweimal für 1 h mit 20%iger Salzsäure bei 100 bis 110° C behandelt und mit Wasser säurefrei gewaschen. Nach dem Trocknen bei etwa 110 bis 120° C wurde in den Zylindern ein Aluminiumoxidgehalt von 2,9% gefunden, die spezifische Oberfläche betrug 230 m²/g.

Diese Formkörper wurden sodann mit 60 Gew.%iger Phosphorsäure überflutet, die 2 h einwirkte, danach wurde erneut bei etwa 110 bis 120° C getrocknet. Diese so behandelten Zylinder hatten einen $H_3PO_4$-Gehalt von 38 Gew.%.

Beim Einsatz dieses so fertiggestellten Hydratationskatalysators zur Synthese von Ethanol aus Ethylen und Wasser in der Gasphase konnte eine Katalysatorausbeute von 105 g Ethanol/h und einer Katalysatorschüttung erzielt werden im Vergleich zu 80 g Ethanol/h und einer Katalysatorschüttung, die bei Vorgehen gemäss DE-A Nr. 1156772 unter gleichen Reaktionsbedingungen erzielt werden.

*Beispiel 2:*

Ein gemahlener natürlicher Rohton, der aufgrund einer Laboruntersuchung so ausgewählt wurde, dass bei einer 1-stündigen Behandlung mit 20%iger Salzsäure bei 82° C nicht mehr als 5 g $K_2O$/kg eingesetzte Trockensubstanz extrahiert werden, wurde 1 h lang mit 20%iger Salzsäure auf 82° C erhitzt, wobei die Säuremenge so bemessen wurde, dass auf 1 kg Ton 8,4 mol HCl kamen, säurefrei gewaschen und getrocknet. Dabei wurde ein Material mit einem Restaluminiumoxidgehalt von 14% und einer spezifischen Oberfläche von 270 m²/g erhalten. Durch Zugabe von 3% des Gewichtes der getrockneten Substanz an gefällter Tonerde wurde der $Al_2O_3$-Gehalt auf 17% eingestellt.

Nach Anfeuchten mit 30% Wasser auf die Gesamtmenge bezogen (das ist Zugabe von 43% der Trockensubstanz als Wasser) wurde das Material zu Kugeln von 3 mm Durchmesser verpresst und verfestigt durch 3 h langes Erhitzen auf 700° C.

Die so erhaltenen geformten Kontaktkörper wurden insgesamt zweimal für 1 h mit 20%iger Salzsäure bei 100 bis 110° C behandelt und mit Wasser säurefrei gewaschen. Nach dem Trocknen bei etwa 110 bis 120° C wurde in den Kugeln ein Aluminiumoxidgehalt von 1,8% gefunden, die spezifische Oberfläche betrug 180 m²/g. Diese Formkörper wurden sodann mit 60 Gew.%iger Phosphorsäure überflutet, die 2 h einwirkte, danach wurde erneut bei etwa 110 bis 120° C getrocknet. Diese so behandelten Kugeln hatten einen $H_3PO_4$-Gehalt von 37 Gew.%.

Beim Einsatz dieses so fertiggestellten Hydratationskatalysators zur Synthese von Ethanol aus Ethylen und Wasser in der Gasphase konnte eine Katalysatorausbeute von 110 g Ethanol/h und einer Katalysatorschüttung erzielt werden. Trotz der geringeren spezifischen Oberfläche in den ausgelaugten Formkörpern ist die Katalysatoraktivität etwas höher als im Beispiel 1, weil die Schüttkörperoberfläche durch den kleineren Kugeldurchmesser grösser geworden ist.

*Beispiel 3:*

Eine hochaktive Bleicherde mit einer spezifischen Oberfläche von 260 m²/g und folgender chemischer Analyse: 72,5% $SiO_2$, 14,0% $Al_2O_3$, 4,0% $Fe_2O_3$, 1,5% MgO, 0,8% CaO, 7,2% Glühverlust, $K_2O < 0,1\%$ wurde mit 30% Wasser auf die Gesamtmenge bezogen (das ist Zugabe von 43% der Trockensubstanz als Wasser) angefeuchtet, zu Kugeln von 4 mm Durchmesser verpresst und durch 3 h langes Erhitzen auf 600° C verfestigt.

Die so erhaltenen geformten Kontaktkörper wurden insgesamt zweimal für 1 h mit 20%iger Salzsäure bei 100 bis 110° C behandelt und mit Wasser säurefrei gewaschen. Nach dem Trocknen bei etwa 110 bis 120° C wurde in den Kugeln ein Aluminiumoxidgehalt von 1,5% gefunden, die spezifische Oberfläche betrug 190 m²/g. Diese Formkörper wurden sodann mit 60 Gew.%iger Phosphorsäure überflutet, die 2 h einwirkte, danach wurde erneut bei etwa 110 bis 120° C getrocknet. Diese so behandelten Kugeln hatten einen $H_3PO_4$-Gehalt von 38 Gew.%.

Beim Einsatz dieses so fertiggestellten Hydratationskatalysators zur Synthese von Ethanol aus Ethylen und Wasser in der Gasphase konnte eine Katalysatorausbeute von 110 g Ethanol/h und einer Katalysatorschüttung erzielt werden.

Beim Einsatz dieses Materials zur Synthese von Isopropylalkohol aus Propylen und Wasser in der Gasphase konnte eine Katalysatorausbeute von 300 g Isopropylalkohol/h und einer Katalysatorschüttung erzielt werden, unter gleichen Reaktionsbedingungen mit Material nach DE-A Nr. 1156772 wurden 220 g Isopropylalkohol/h und eine Katalysatorschüttung erhalten.

**Patentansprüche**

1. Verfahren zur Herstellung eines Katalysators für die Hydratation von Olefinen mit 2 bis 3 C-Atomen zu den entsprechenden Alkoholen, durch Behandlung eines geformten Trägers aus montmorillonithaltigem Ton mit Säure, bis er einen $Al_2O_3$-Gehalt von 1 bis 5% aufweist, Tränken des Trägers mit Phosphorsäure und Trocknen, dadurch gekennzeichnet, dass man als Träger einen Ton verwendet, der mit nicht mehr als 3% Begleitmineralien, wie Quarz, Feldspat und Glimmer, verunreinigt ist und bis 0,5% $K_2O$ enthalten darf, diesen Ton vor der Verformung bereits mit Säure behandelt, bis er einen $Al_2O_3$-Gehalt von 13 bis 18 Gew.% aufweist (1.Stufe), gegebenenfalls durch Zugabe von gefällter Tonerde den $Al_2O_3$-Gehalt auf 16 bis 18 Gew.% einstellt, dann bei einem Gesamtwassergehalt von 20 bis 35% durch Pressen formt, bei 500 bis 800° C calciniert und anschliessend in bekannter Weise unter nochmaliger Säurebehandlung (2.Stufe) weiterverarbeitet.

2. Verfahren nach Anspruch 1, dadurch gekennzeichnet, dass auch andere Mineralien der Montmorillonitgruppe, die kein Kalium, aber das Montmorillonitkristallgitter enthalten, eingesetzt werden.

3. Verfahren zur Herstellung eines Katalysators nach den Ansprüchen 1 und 2, dadurch gekennzeichnet, dass man als Ausgangsmaterial eine bereits einmal säurebehandelte (1.Stufe) hochaktive Bleicherde — hergestellt aus einem Ton mit hohem Montmorillonitgehalt — verwendet, die weniger als 0,1% $K_2O$ enthält und deren Gewichtsverhältnis $(Al_2O_3 + Fe_2O_3) : SiO_2$ 1 : 3,5 bis 1 : 4,5 beträgt.

4. Verfahren nach Anspruch 1 bis 3, dadurch gekennzeichnet, dass man zur Vergrösserung der katalytisch wirksamen Oberfläche Kontaktkörper (Kugeln, Zylinder, Tabletten) mit einem Durchmesser in den Abmessungen zwischen 2 und 5 mm, bevorzugt 2 bis 3 mm, formt.

5. Katalysator zur Hydratation von Olefinen zu Alkoholen, hergestellt nach den Ansprüchen 1 bis 4.

## Revendications

1. Procédé de fabrication d'un catalyseur pour l'hydratation des oléfines ayant 2 à 3 atomes de carbone en les alcools correspondants, par traitement d'un support moulé en argile contenant de la montmorillonite avec un acide, jusqu'à ce qu'il présente une teneur en $Al_2O_3$ de 1 à 5%, par imprégnation du support avec de l'acide phosphorique et par séchage, caractérisé en ce qu'on utilise comme support une argile qui n'est pas contaminée par plus de 3% de minéraux accompagnateurs tels que le quartz, le feldspath et le mica, et qui peut contenir jusqu'à 0,5% de $K_2O$, on traite cette argile déjà avant moulage avec un acide jusqu'à ce qu'elle présente une teneur en $Al_2O_3$ de 13 à 18% en poids (1er stade), en ce que, éventuellement par une addition d'alumine précipitée, on règle la teneur en $Al_2O_3$ à 16 à 18% en poids, puis on moule par compression à une teneur globale en eau de 20 à 35%, on calcine à 500 à 800° C, puis on poursuit le traitement de manière connue avec nouveau traitement à l'acide (2e stade).

2. Procédé selon la revendication 1, caractérisé en ce qu'on utilise aussi d'autres minéraux du groupe de la montmorillonite, lesquels ne contiennent pas de potassium mais contiennent le réseau cristallin de la montmorillonite.

3. Procédé de fabrication d'un catalyseur selon les revendications 1 et 2, caractérisé en ce qu'on utilise comme matière de départ une terre décolorante très active déjà traitée une fois à l'acide (1er stade), qui a été préparée à partir d'une argile à teneur élevée en montmorillonite et qui contient moins de 0,1% de $K_2O$ et dont le rapport pondéral $(Al_2O_3 + Fe_2O_3) : SiO_2$ s'élève à 1 : 3,5 jusqu'à 1 : 4,5.

4. Procédé selon les revendications 1 à 3, caractérisé en ce que, pour l'agrandissement de la surface catalytiquement active, on moule des corps de contact (billes, cylindres, tablettes) ayant un diamètre dans les dimensions comprises entre 2 et 5 mm, de préférence de 2 à 3 mm.

5. Catalyseur pour l'hydratation des oléfines en alcools, préparé selon les revendications 1 à 4.

## Claims

1. Process for the production of a catalyst for the hydration of olefins with 2 to 3 C-atoms to the respective alcohols, by treatment of a formed carrier of montmorillonite-containing clay with acid, until it shows an $Al_2O_3$-content of 1 to 5%, impregnation of the carrier with phosphoric acid and drying, characterized in that a clay is used as carrier, which is contaminated by not more than 3% accompanying materials, such as quartz, feldspar and mica, and which may contain up to 0,5% $K_2O$, this clay being already treated befor forming with acid, until it shows an $Al_2O_3$-content of 13 to 18 wt.% (1st stage), if necessary, by adding of precipitated pure clay the $Al_2O_3$-content is adjusted to 16 to 18 wt.%, then molded by squeezing with a total water content of 20 to 35%, calcined at 500 to 800° C and subsequently worked in known manner by repeated acid treatment (2nd stage).

2. Process according to claim 1, characterized in that also other minerals of the montmorillonite group are used which do not contain potassium but the montmorillonite crystal lattice.

3. Process for the production of a catalyst according to claims 1 and 2, characterized in that as starting material a highly active bleaching earth, already once treated with acid (1st stage), produced from a clay with a high montmorillonite content, which contains less than 0,1% $K_2O$ and the weight ratio amounts to $(Al_2O_3 + Fe_2O_3)$ $SiO_2$ 1 : 3,5 to 1 : 4,5.

4. Process according to claims 1 to 3, characterized in that contact bodies (spheres, cylinders, tablets) with a diameter having dimensions between 2 and 5 mm, preferably 2 to 3 mm, are used in order to enlarge the catalytically effective surface.

5. Catalyst for the hydration of olefins to alcohols produced according to the claims 1 to 4.